# EUROPEAN PATENT SPECIFICATION

(11) **EP 2 773 959 B1**
(45) Date of publication and mention of the grant of the patent: **10.04.2019**
(21) Application number: 12844702.6
(22) Date of filing: 05.11.2012
(51) Int. Cl.: G01N 33/559, C07K 1/26, C07K 1/28, G01N 27/447, C07K 1/36

(54) **PROTEIN FRACTIONATION BASED ON ISOELECTRIC FOCUSING**
PROTEINFRAKTIONIERUNG DURCH ISOELEKTRISCHE FOKUSSIERUNG
FRACTIONNEMENT DE PROTÉINES EN FONCTION DE LEUR POINT ISOÉLECTRIQUE

(30) Priority: 04.11.2011 US 201161555564 P; 04.11.2011 US 201161555592 P; 04.11.2011 US 201161555674 P
(43) Date of publication of application: 10.09.2014
(73) Proprietor: Bio-Rad Laboratories, Inc., Hercules, CA 94547 (US); Technion Research & Development Foundation Ltd., 32000 Haifa (IL)
(72) Inventor: PAULUS, Aran, Hercules California 94547 (US); DIGES, Camille, Hercules California 94547 (US); BOGOEV, Roumen, Hercules California 94547 (US); BANDHAKAVI, Sricharan, Hercules California 94547 (US); HAHN-WINDGASSEN, Annett, Hercules California 94547 (US); POSCH, Anton, 85567 Grafting (DE); BROD, Elad, Haifa 32000 (IL); SIVAN, Uri, Haifa 32000 (IL)
(74) Representative: dompatent von Kreisler Selting Werner - Partnerschaft von Patent- und Rechtsanwälten mbB
(86) International application number: PCT/US2012/063601
(87) International publication number: WO 2013/067529

(56) References cited:
- EP-A2- 1 748 340
- WO-A1-2006/063625
- WO-A1-2007/051492
- WO-A1-2011/021195
- WO-A2-03/019172
- WO-A2-2009/027970
- WO-A2-2010/048173
- WO-A2-2011/021196
- US-A- 5 160 594
- US-A- 5 773 645
- US-A1- 2009 101 491
- US-A1- 2010 155 243
- US-A1- 2010 307 920
- US-A1- 2012 138 468
- US-A1- 2012 145 548

## Description

### BACKGROUND

Current isoelectric focusing based protein/peptide fractionation technologies suffer from at least two shortcomings. First, samples are separated over a fixed or limited pH range resulting in non-optimal fractionation of various samples. Second, pH gradients required for sample fractionation are established via chemicals (ampholytes) resulting in contamination of fractionated samples with chemicals and (potential) interference of downstream analysis.

WO 2006/063625 discloses a method for separating proteins dependent on their isoelectric point.

### BRIEF SUMMARY OF THE INVENTION

In some embodiments, the present invention provides a device for separating a plurality of molecular analytes according to both isoelectric points and electrophoretic mobility within one dimension, the device comprising a chamber for containing a solution having a plurality of molecular analytes, wherein the chamber contains a sieving medium suitable for electrophoresis; an electrical source for applying an electric field along an axis in the chamber; a one or more ion sources for establishing a pH gradient along said axis in said chamber by injecting ion flows, capable of forming one or more pH steps in a pH gradient, wherein each of said ion sources is a proton injector or a hydroxide injector separated from said chamber by a bipolar membrane; and a controller which operates said one or more ion sources to adjust the pH gradient so as to induce migration and capturing of the molecular analytes separately along the axis.

In some embodiments, the present invention provides a method of separating one or more target protein from a sample, the method comprising providing into the chamber of the device of the invention a sample comprising a mixture of proteins including one or more target protein; submitting the proteins in the chamber to electrophoresis; and subsequently generating a pH gradient in the chamber with a proton and/or hydroxide injector, thereby positioning proteins in the chamber within one dimension based on both the isoelectric point (pI) and the electrophoretic mobility of the proteins.

In some embodiments, the method further comprises collecting the one or more target protein.

In some embodiments, the chamber of the device of the present invention contains one or more ports in fluid communication with the chamber and movably positioned in the chamber to capture a desired analyte based on the analyte's position.

### BRIEF DESCRIPTION OF THE DRAWINGS

Figures 1A and 1B illustrate a proton and hydroxide injector, respectively, comprising a small compartment adjacent to the channel/chamber, with a Pt electrode dipped inside it, and a bipolar membrane separating the compartment from the channel/chamber.
Figure 2 illustrates possible electrolytes and their interaction with a proton injector. It will be appreciated that similar interactions can be provided using a hydroxide injector.
Figure 3 illustrates an arrangement for a proton/hydroxide injector device.

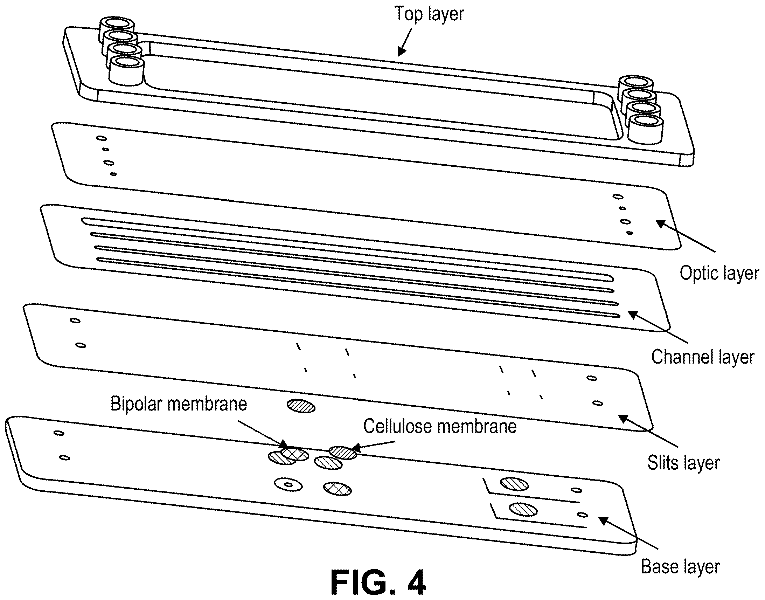

Figure 4 illustrates an example of an integrated disposable channel for use in a proton injector or hydroxide injector device. Slits for fluid contact to proton/hydroxide injector compartments can be arranged as desired. For example, slits in the chamber may be 1-1000 µm, and in some cases, about 100 µm. The number and size of slits can be designed to generate step-wise pH gradients as desired. Cellulose, or other hydrophilic, membranes, for example, as shown in Figure 4 are optional, and function to cover unused slits and/or can optionally cover bipolar membranes to the extent sample components have affinity to the bipolar membrane. Instead of hydrophilic membranes, hydrophilic coatings may be used to cover the bipolar membranes and prevent binding of the sample components to it. Further slits can be used to extract and inject samples to the channel.
Figures 5A-C illustrate generation of a pH step gradient and isolation of target molecules with the gradient. In the figure, a bipolar membrane (2) generates a large pH differential, thereby focusing unwanted components (1) of the sample away from the target analyte. A second bipolar membrane (4) generates a small pH differential centered at the pI for the target analyte (3). The target analyte (3) can optionally be collected in a channel (5) in the chamber.
Figure 6 illustrates generation of a pH step gradient and isolation of multiple target molecules with the gradient. This can be used for subsequent application to electrophoresis or other applications.
Figure 7 illustrates generation of a pH step gradient and isolation of target molecules with the gradient. This can be used, for example, for protein clean-up, capture and direct injection into mass spectrometer (MS), or other detection methods.
Figures 8A-D illustrate isolation and collection of a target molecule using pH gradients.
Figure 9 illustrates a chamber or channel as described herein being adapted for delivery of analytes to a mass spectrometer (Figure 9A) or to a light source (Figure 9B).
Figures 10A-C illustrate digital pH analytical separation of a target molecule.
Figure 11 illustrates separation of molecules based on two separate criteria within one dimension. Figure 11 shows the same channel running top to bottom in the figure at four time points (a), (b), (c), and (d). The numbers to the left of the channel indicate pH, with a proton or hydroxide injector shown across the channel at each position at which pH is indicated. Sample components (111, 112, 113, 114, 115) are shown moving electrophoretically over time ((a), (b), (c), and (d))
Figure 12 illustrates separation of molecules by pI and a second separate criteria in another dimension.
Figure 13 illustrates a target protein being separated from contaminants based on pI and subsequently crystallized. The protein is separated from contaminations, moved to a chamber, optionally with an integrated loop, and left for crystallization, flash frozen, and/or imaged.

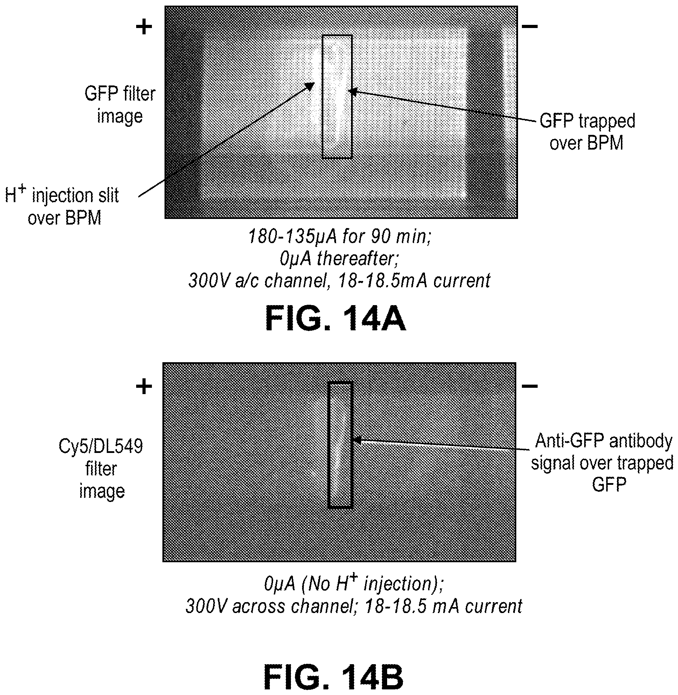

Figure 14A represents green fluorescent protein (GFP) signal following electrophoresis in buffer and 'trapping' via H⁺ injection in the same buffer-filled chamber. Figure 14B illustrates CY5 signal following precipitation/'trapping' of the GFP and introduction of a CY5-labeled anti-GFP antibody. Buffer used is 4 mM Sodium Citrate, 4 mM Sodium Phosphate (dibasic), 4 mM Sodium Pyrophosphate, and 13 mM Sodium Sulfate, pH 8.5.

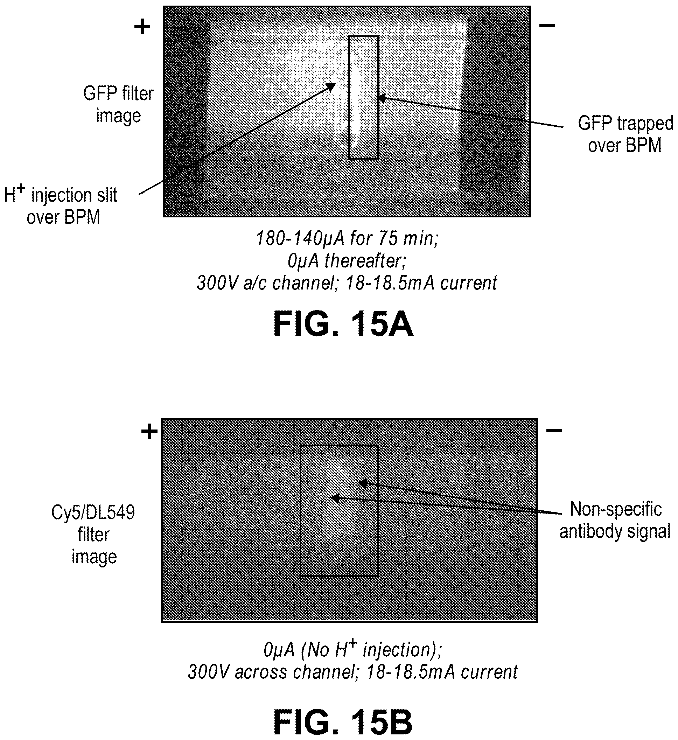

Figure 15A represents green fluorescent protein (GFP) signal following electrophoresis in buffer and 'trapping' via H⁺ injection in the same buffer-filled chamber. Figure 15B illustrates CY5 signal following precipitation/'trapping' of the GFP and introduction of a CY5-labeled anti-rabbit (non-specific) antibody. Buffer used is 4 mM Sodium Citrate, 4 mM Sodium Phosphate (dibasic), 4mM Sodium Pyrophosphate, and 13 mM Sodium Sulfate, pH 8.5.

### DETAILED DESCRIPTION OF THE INVENTION

Described in more detail herein are methods and apparatuses that allow for detection, purification, and/or isolation of target molecules (e.g., proteins, peptides, nucleic acids) from samples in a chamber in an apparatus optionally using 1) electrical fields to move the targets combined with 2) electronic control of pH of solution in sub-areas of the chamber using proton or hydroxide injectors. The methods take advantage of the pH-dependence of charge of targets, for example allowing for localization of charged targets to a particular sub-area by setting the pH of solution in proximity to the sub-area to a pH at or close to the pI of the target of interest. At a target's pI, the target becomes uncharged and therefore does not move further in an electric field. A number of examples using this aspect are described below.

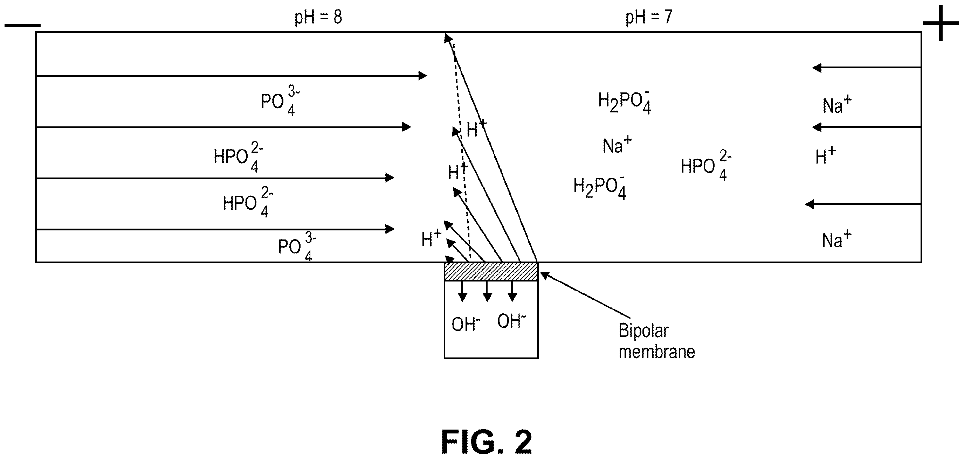

The apparatus can have a variety of configurations. In some aspects, the apparatus comprises at least one chamber having a first and second electrode, which allow for moving a charged target in an electric field. The chamber can comprise one or more (e.g., 1, 2, 3, 4, 5, or more) proton or hydroxide injector separated from the chamber by a bipolar membrane, wherein the injector comprises an electrode, thereby allowing for electro-hydrolysis of water molecules. *See*, *e.g.*, Figure 2. The terms "chamber" and "channel" are used synonymously. The terms encompass containers that are considerably (e.g., 10x, 100x, 1000x) longer than wide, which allow for multiple injectors along the long axis of the chamber.

Chambers of the following dimensions have been constructed:

| **Channel L/H/W in mm** | **Slit L/H/W In mm** | **Material** | **Channel volume (Vc; in µl)** | **Slit volume in µl (Vs; in µl)** |
|---|---|---|---|---|
| 90 X 0.3 X 3 | 3 X 0.5 X 0.3 | Glass/PMMA | 81 | 0.45 |
| 36 X 0.2 X 1 | 1 X 0.2 X 0.2 | COC | 7.2 | 0.04 |
| 221 X 0.25 X 1 | 1 X 0.25 X 0.2 | PMMA | 55 | 0.05 |
| 36 X 0.15 X .5 | .5 X 0.1 X 0.1 | PMMA | 2.7 | 0.005 |
| 33.6 X 0.25 X 1 | 1 X 0.25 X 0.23 | PMMA | 8.4 | 0.0575 |
| 221 X 0.25 X 1 | 1 X 0.25 X 0.2 | PMMA | 55 | 0.05 |

| | | | | |
|---|---|---|---|---|
| "Slits" refer to the size of the hole in the chamber through which the proton or hydroxide injector is connected to the chamber. A bipolar membrane at the slit divides the chamber from the injector. | | | | |

The orientation of the electrodes (i.e., which is a cathode and which an anode) will depend on the charge of the molecules to be moved in the solution and the direction the molecules are to be moved. For example, a positively-charged molecule moves towards a cathode and a negatively-charged molecule moves towards an anode when an electrical voltage difference is present through the solution in the chamber between the cathode to the anode.

Generally, the electrodes should be oriented so that they are as close to each other as possible, i.e., directly across from each other. While other configurations are contemplated and possible, voltage and resistance increases as a function of distance.

Electrodes in the chamber can in some circumstances interfere and/or bind target molecules (e.g., protein) in the chamber. Thus, the electrodes may be separated from the chamber by a membrane or gel, thereby preventing target molecules from binding the electrodes.

The size and shape of the chamber can vary. While the chamber is depicted as a tube or channel (i.e., longer between the electrodes than across other axis), other configurations are also possible.

A proton or hydroxide "injector " refers to one or more compartments, separated from a sub-chamber or other vessel (e.g., such as a reservoir), by a hole or "slit" and divided by a bipolar membrane(s), wherein the compartment(s) contain an electrode(s). Depending on the orientation of the electric field (e.g., orientation of the anode and cathode) in the compartment(s), the compartment(s) can be designed to inject protons or hydroxide ions through the bipolar membrane(s) and into the adjacent chamber.

By controlling the current and configuration, one can thereby control the pH of solution in the chamber in proximity to the proton or hydroxide injector. Generally, it can be desirable to increase the surface area of the bipolar membrane as this allows for decreased electrical resistance.

The membrane(s) "divides" the compartments from the chamber by forming a barrier that separates solution in a compartment from the chamber, e.g., at least to the level of solution in the chamber. For example, when the chamber is open at the top (or alternatively, has a top cover that can be removed), the membrane(s) can be designed to completely divide a compartment from the chamber at least up to the level of solution in the chamber and/or compartment, or to a level designated as a maximum for solution loading. As desired, the membranes can be designed to be higher than the solution level so as to avoid accidental transfer (e.g., splashing) from one portion to another. If desired, the membranes can be "framed" by a solid material (e.g., plastic) or otherwise anchored between the chamber and the compartment.

The electrodes can be formed from any conducting or semi-conducting substance. For example, one or more electrode may comprise a metal. The metal may be zinc, copper, or platinum. For example, the electrodes can be platinum or can be platinum-plated. Generally, maximal surface area for electrodes is desirable. A flattened electrode, for example, provides more surface area than a wire.

International Patent Application Publication WO2009/027970 describes methods and devices (i.e., proton or hydroxide injectors) useful in producing local concentrations of protons or hydroxide ions, proton or hydroxide concentration gradients, and desired proton or hydroxide concentration topographies in an environment, such as an electrolyte solution and a gel. International Patent Application Publication WO2011/021195 and WO2011/021196 describe methods and devices for isoelectric focusing using proton/hydroxide injectors and also describes display of data.

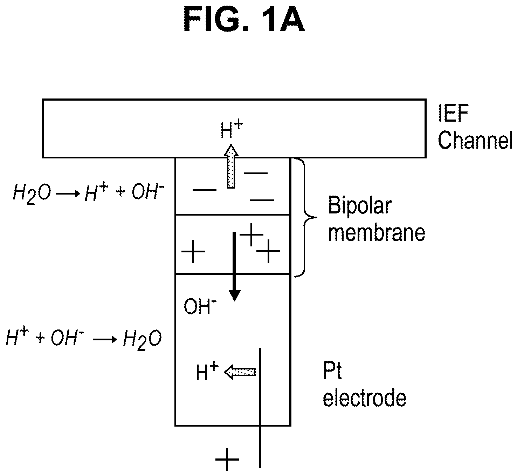

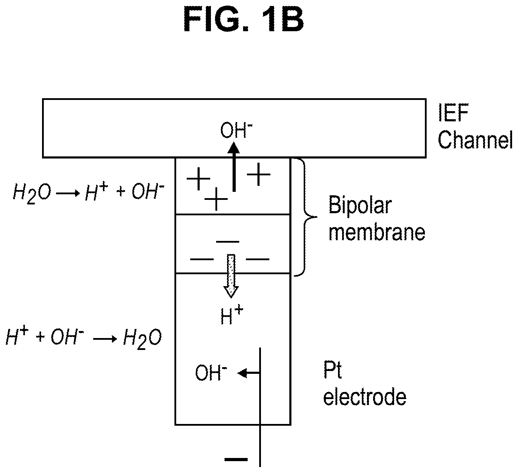

Proton/hydroxide injector technology can be used to affect the pH of the solution in a chamber, or at least the solution in the chamber in proximity to the injector. Briefly, in, the proton/hydroxide injector may comprise a compartment adjacent to the apparatus chamber, with an electrode inside the compartment, and a bipolar membrane separating the compartment from the channel. *See*, *e.g.*, Figures 1A-1B. A bipolar membrane is an ionexchange membrane having a structure in which a cation-exchange membrane and an anion-exchange membrane are joined together, and allows for water molecules to be split into protons and hydroxide ions. Voltage applied between the compartment and the channel divided by the bipolar membrane leads to water splitting and injection of protons or hydroxide ions into the channel. Some advantages of this technology can include, for example, bubble-free water hydrolysis and injection of generated ions directly to the channel, allowing short response time (e.g., if desired, below 1 min).

By applying the appropriate voltage to the electrodes in the chamber an electric field across the solution in the chamber is generated and charged molecules move accordingly. The charged molecules can be added in proximity to the anode or cathode in the chamber (in which the pH is controlled at least in part by a proton injector or a hydroxide injector), and subsequently the voltage is applied, thereby delivering the charged molecule to a desired position in the chamber at a time determined by the user.

The direction of movement of the molecule will depend on the charge of the molecule and the polarity of the applied voltage.

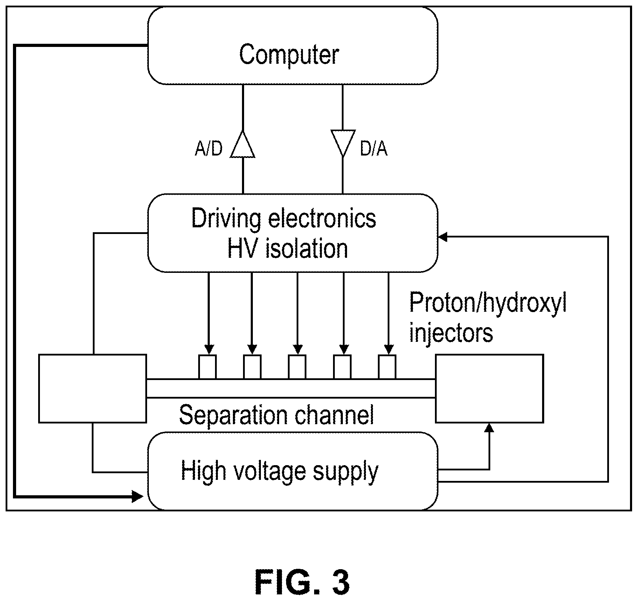

Systems incorporating the apparatus are disclosed. Systems can include, for example, a power supply and power regulator to control current and/or voltage to electrodes in the chamber and/or injectors. *See*, *e.g.*, Figure 3. Pumps for regulating flow of liquids, a mechanism for stirring or mixing solution in the chamber, and heating or cooling units can be included. The system may include a pH and/or conductivity probe in the chamber. Generally, it can be desirable to place the probe at a distance from the electric field lines between electrodes to improve readings.

### Methods and devices for detecting and collecting analytes fractionated based on pI

Dynamically adjustable pH 'step/s' spanning the pH range of ∼2-12 (can be further extended or contracted as needed) can be generated within a chamber filled with suitable buffers using proton and/or hydroxide injectors as described herein. Use of proton or hydroxide injectors to control pH as described herein can be designed such that target analytes reach their pI in only minutes, for example, in less than, e.g., 10, 20, or 30 min.

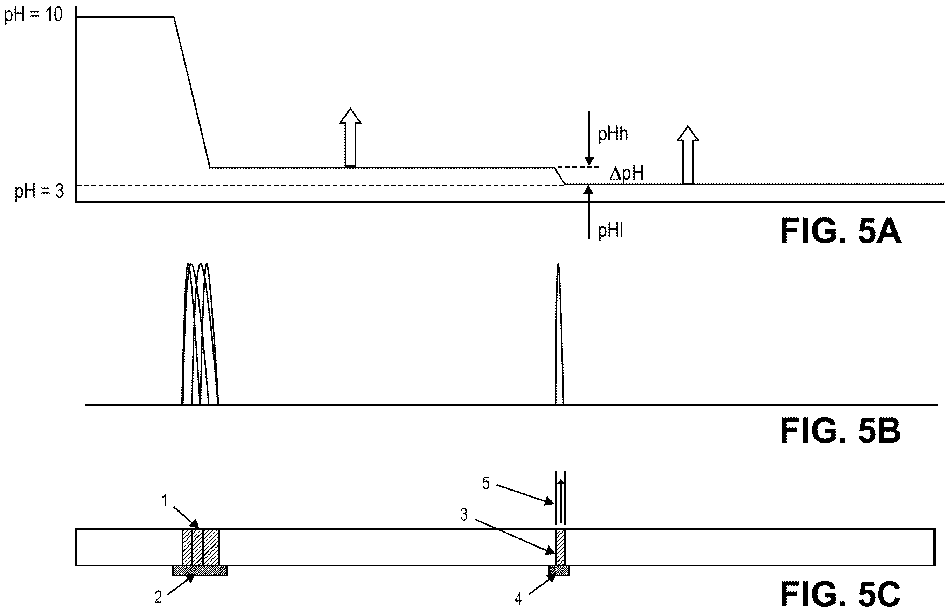

An example of such a gradient is displayed in Figure 5A. Figure 5A illustrates a relatively large difference in pH between two regions of the chamber (left side) being used to capture a majority of analytes having a pI within the pH range. To the right a smaller pH range (designed specifically to span a particular target analyte pI) is shown, thereby isolating the target analyte without significant amounts of other components of the sample. Complex mixtures of suitably buffered analytes (including proteins and/or peptides) will be submitted to an electric field within the chamber so as to 'capture' proteins (or peptides) at their respective isoelectric points (pI) in either a single pH step (see Figure 5B) or multiple pH 'step/s' spanning the desired pH range. Subsequently, when collection of the purified target is desired, ampholyte-free, charged species can be released from the chamber towards a harvesting chamber for collection and downstream analysis. *See*, *e.g.* Figure 5C. Movement of the purified target from the chamber into collection can be achieved, for example, by physical pumping, electro osmotic pumping, or electronic adjustment of H⁺/OH⁻ generation at (each) gradient 'step'. *See*, *e.g.* Figure 5C. This approach can allow for optimized fractionation of various protein/peptide samples (via adjusting protein/peptide capture and release in a sample-dependent manner) or other types of samples (e.g., nucleic acids or other) without contamination by chemical ampholytes that occur in standard isoelectric focusing.

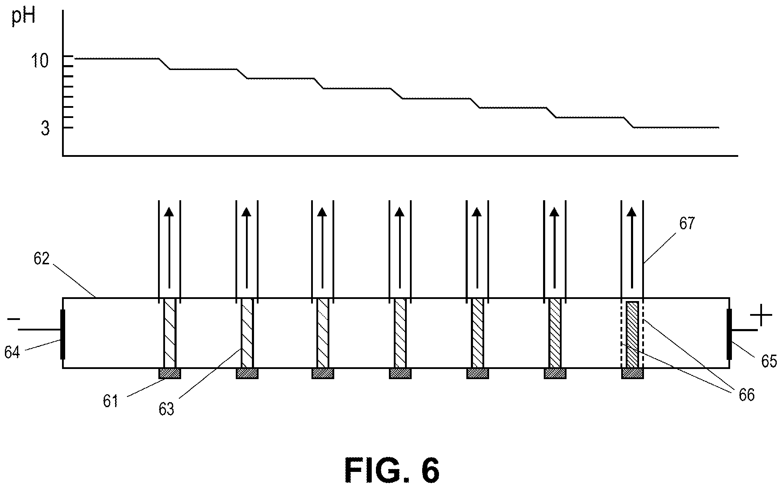

As shown in Figure 6 multiple bipolar membranes (61) may be placed directly under the slits in a channel (62), also referred to herein as a "chamber." The separation channel can be filled with a suitable buffer. Either protons or hydroxide ions are injected by each bipolar membrane to create a step gradient as shown on the pH graph (Figure 6). The peptides or proteins (63) focus in the steps corresponding to their pI by applying an orthogonal electrical field through electrodes (64) and (65). Optional permeable membranes or screens (66) can be used to create chambers where the proteins or peptides are focused. After the focusing is completed the target analytes (e.g., peptides or proteins) are harvested through harvesting ports (67) in fluid communication with the channel, allowing for collection of target analytes having a specific pI. Collection ports can be of a diameter useful for collection. The collection ports may be 100 µmor less in diameter, e.g., 1-100 µmin diameter.

The technology may be used to address two issues: the cleanup (e.g., removal or reduction of one or more contaminant) and/or concentration of a protein of interest.

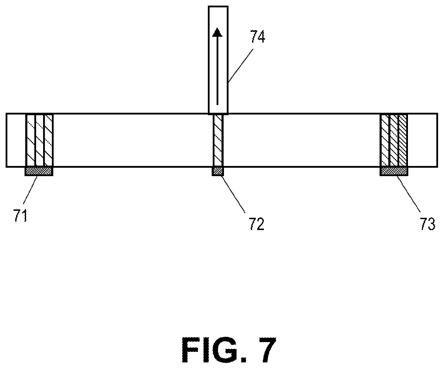

As shown in Figure 7, the protein sample may be separated into at least three fractions:
- The proteins with pI higher than the pI of the target protein (or other target analyte) are isoelectrically focused in the region of bipolar membrane (71) where a pH step encompassing pH higher than the pI of the antigen is created.
- The protein of interest is focused in the region of bipolar membrane (72) by creating a narrow pH range step encompassing the pI of the protein of interest.
- The proteins with pI lower than the pI of the target protein are isoelectrically focused in the region of bipolar membrane (73) where a pH step with range below the pI of the protein of interest is created.

In this way, the protein of interest can be separated (purified) from the other proteins and other contaminants and concentrated in the area close to the harvesting channel. Subsequently the protein of interest can be harvested via a harvesting port or through harvesting channel (74). The harvesting can be accomplished using, for example, liquid flow or electrophoresis.

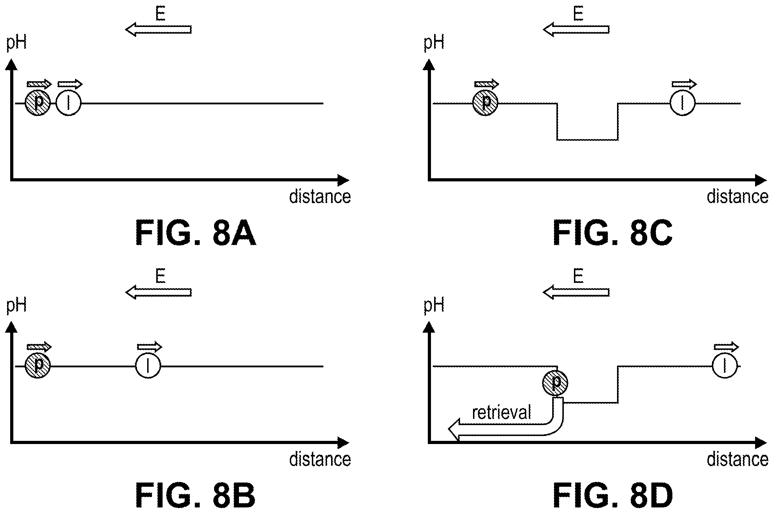

Figures 8A-D illustrate protein cleanup and capture. Here, electronically generating a pH step gradient is exploited for protein purification. Generally, purifying specific macromolecules from a mixture is most efficiently achieved when the process of purification is based on some known property of the macromolecule (like mass, mobility, affinity). Such is the case in affinity columns, electrophoresis, ion exchanger column and many other purification techniques. As described herein, the relevant properties of the molecule include their isoelectric point (pI) and mobility under electric field.

In the purification apparatus, a pH step may be created in a channel to which the protein of interest (POI) is inserted together with some impurities, e.g., other components of the sample. The pH step is designed according to the pI of the POI and the surrounding impurities so that the pI of the former will fall in the range of the step while the pI of the latter will not. In this way the protein will focus in a sharp band as shown in Figure 7 while the latter will continue migrating towards the end of the channel. This procedure is very simple in the case were the impurities and POI have distant isoelectric points or in the case were the impurities lack a pI altogether. In the case were the pI's are close, the difference in mobility can be used as one of several criteria for separation.

An example is given in Figure 8. The POI and some impurities, marked "P" and "I" respectively, migrate under constant pH conditions (Figure 8A). In case the impurities are faster than the protein (most likely this is the case when the impurities are small molecules or short peptides) a gap develops between the two (Figure 8B). When this gap is large enough, the constant pH profile is changed to one with an acidic depression, as in Figure 8C, which causes the protein to focus in a pH step, while the impurities continue to migrate towards the end of the channel (Figure 8D). In addition to the purification power, the apparatus may have a retrieval system, e.g., for further analysis of the POI.

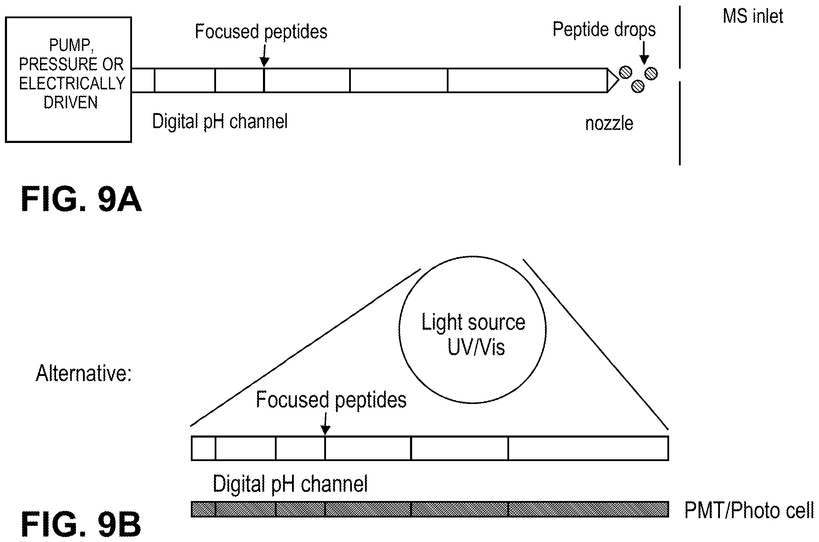

Figure 9 shows aspects of the technology described herein in which a simple or complex mixture (sample) of proteins or peptides is submitted to isoelectric focusing via pH step gradients and one or more target analyte in the sample is detected. As shown in Figure 9A, the end of a chamber in which the pH gradients are set can be fitted with a nozzle or other device for delivering isoelectrically purified portions directly to a mass spectrometer (MS). This allows for delivery of a simplified sample (starting from the original mixture of higher complexity) to the MS device and is free from ampholytes (as would occur in other types of isoelectric focusing and which interfere with MS). Alternatively, as shown in Figure 9B, isoelectrically focused analytes can be detected with other detectors, including an in-line fluorescent detector (for detecting fluorescently-labeled analytes), a light source and a UV light source.

One or more target molecules can be focused based on pI using one or more proton or hydroxide injectors and subsequently submitted to electrophoresis. The pI fractions can be precisely positioned where desired (for example on the top of the second dimension channel) when using a proton/hydroxide injector. In contrast, in isoelectrical focusing (IEF) steady state is achieved and therefore, the bands are not moving through the detector. This means either the detector needs to move along the capillary or the whole capillary needs to be imaged. With electronic control of pH as described herein, the target bands can be delivered to the detector, thereby simplifying design.

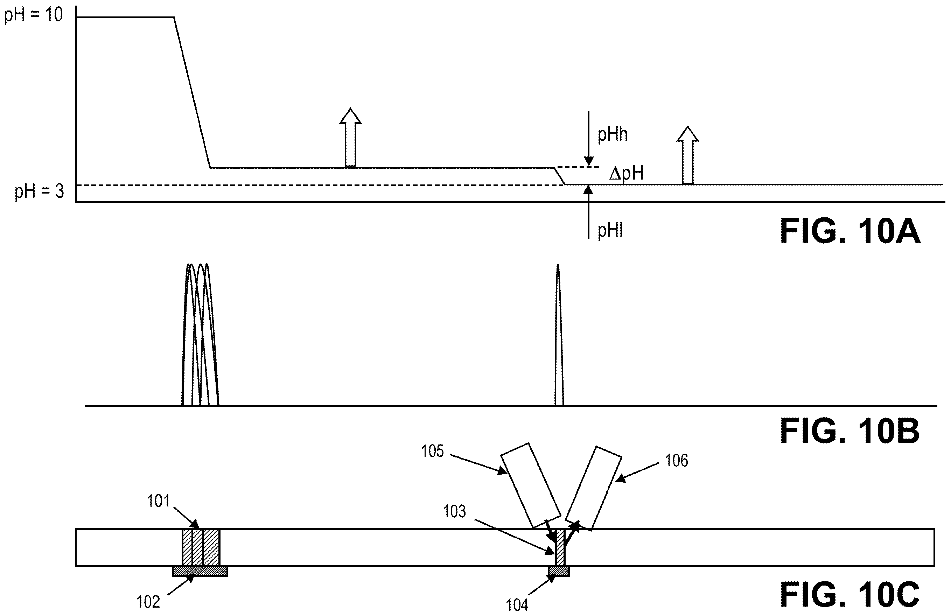

The method of proton injector or hydroxide injector-mediated pH focusing can be used for analytical purposes. In conventional IEF gels or strips, the sample is analyzed in a spatial pattern where proteins focus in their pI based on the location of the pH on the gel. In contrast, employing a proton injector or hydroxide injector, a dynamic map of target (e.g., target protein) quantity v/s pH value can be created. An example is illustrated in Figures 10A-C. The target-containing sample (101) is initially captured at the broad pH step created by a proton injector or hydroxide injector separated from the channel by a bipolar membrane (102). Then the pH in the lower (or upper) range of the step is changed to allow the sample components (103) with pI above the pHl (the low end of the pH in the step) and below pHh (the high end of the pH in the step) to start migrating to the second proton injector or hydroxide injector separated from the channel by a second bipolar membrane (104), e.g., by diffusion or using electrodes in the channel to electrophorese the charged components further down the channel. By increasing the pHl and pHh pH values the sample components can be moved from membrane 102 to membrane 104. By keeping the ΔpH small, the resolution of the methodology can be very high. The target molecules can be detected by any method available, including by using absorbance, fluorescence (conveyed by a dye that attaches to the proteins covalently or non-covalently). As illustrated in Figure 10C, the proteins or other target molecules may be detected by using emitting diode (105) and light capturing diode (106) to detect the light from the excited dye. This method can be used, for example, to determine the relationship between the pI and amount for a complex sample or for a purified protein (for example when looking at charge isoforms).

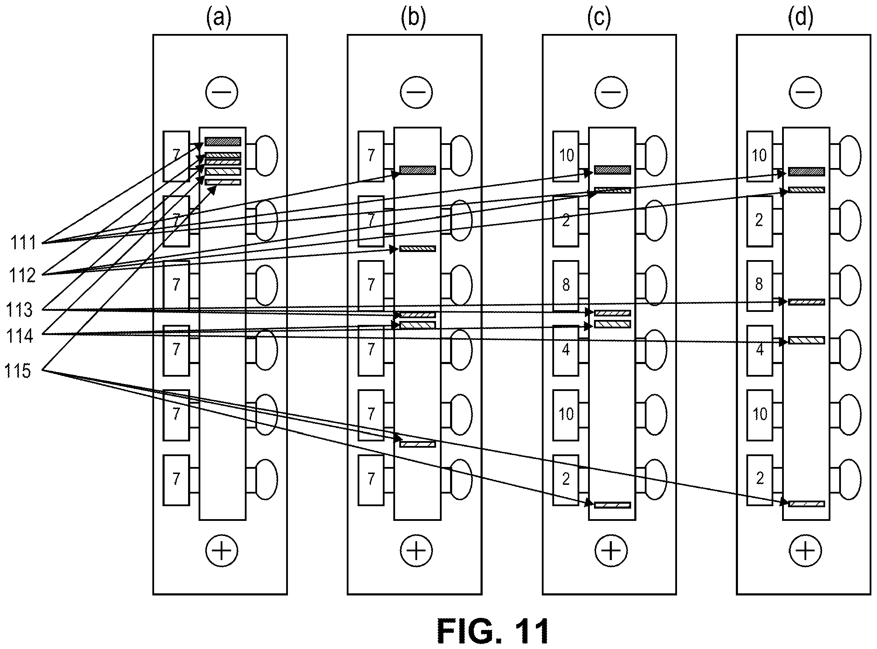

The channel may be filled with a gel rather than a liquid and sample components can be separated by mobility and pI criteria. This technology can be designed, for example as shown in Figure 11. Proton/hydroxide ion injectors facilitate real-time variation of the spatial pH pattern generated by proton and hydroxide ion injection into the separations channel. As a result, the pH gradients used to separate peptides and proteins according to their isoelectric point can be tuned at will, giving way to sequential separation according to isoelectric point and another separation criterion such as electrophoretic mobility or affinity assay. The order of the two separation processes can be chosen at will to guarantee optimal separation.

Common two-dimensional separation gels can be replaced by the disclosed one-dimensional programmable approach. Figure 11 illustrates such separation, first according to the electrophoretic mobility and then according to the isoelectric point. Here, the medium in the chamber will be a gel suitable for electrophoresis (including linear or crosslinked polymers such as for example agarose, linear or crosslinked polyacrylamide and polymers of acrylamide derivatives or other gel types). Imagine for example a mixture of 5 proteins, two of which (114 and 113) characterized by an identical electrophoretic mobility but different isoelectric point, and two (113 and 112) proteins having the same isoelectric point but different electrophoretic mobility. Further, imagine we aim to isolate the 113 protein. In step (a) one sets the channel's pH to 7 and separates the proteins according to their electrophoretic mobility. Since in the specific example of Figure 11, the 113 and 114 proteins have higher mobility compared with protein 111 and 112, they separate after a while from the latter (Figure 11, panel (b)). However, the electrophoretic assay does not separate the 113 protein from the 114 protein because they share a similar electrophoretic mobility. To separate 113 from 114, one tunes the pH profile along the channel in such a way that the 113 protein separates from the 114 one (Figure 11, panel s (c), (d)). At the same time, proper design of the pH profile in other parts of the channels pushes the 111, 112, and 115 proteins away from the 113 and 114 proteins (Figure 11, panels (c), (d)). The outcome of this method is isolation of the desired (113) protein according to two distinct criteria, electrophoretic mobility and isoelectric point, both carried out in the same one-dimensional channel.

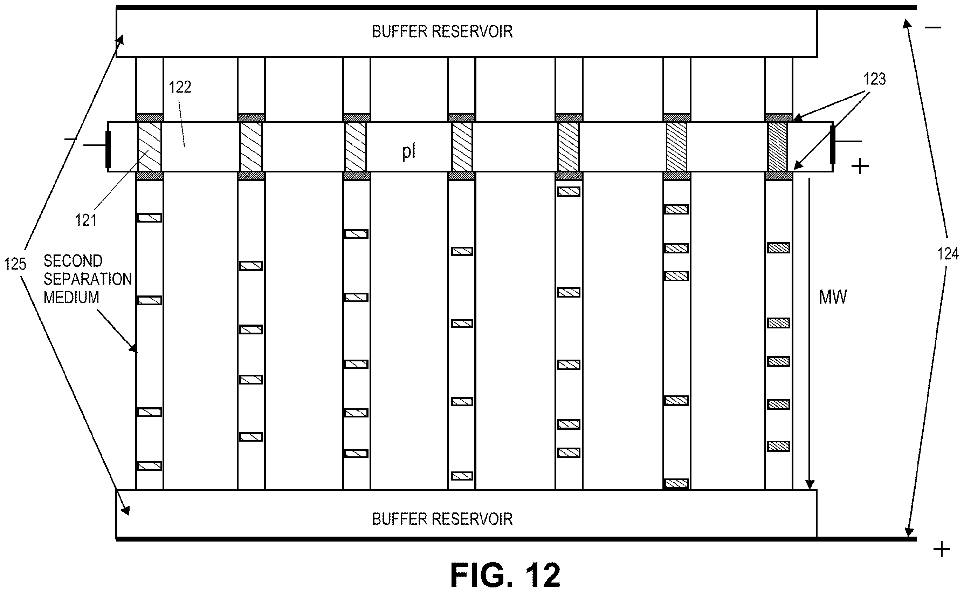

In another option, e.g., as shown in Figure 12, a sample is separated in one dimension by pI and then a second dimension by mobility. Multiple bipolar membranes (121) (and accompanying proton injector or hydroxide injector below or above the plane of the figure) may be incorporated in a channel (122) containing a liquid buffer. By either injecting either protons or hydroxide ions and applying voltage along the channel, step gradient is created and the proteins are focused in the corresponding pH step. After the focusing is complete, the valves (123) on both sides of each (e.g., orthogonal) channel are open and voltage is applied at electrodes (124) and the focused proteins are separated in a second dimension separation. The second dimension separation can be performed by molecular weight, charge or charge and molecular weight. For example, the second dimension may comprise electrophoresis, including SDS-PAGE or native-PAGE separations. The separation media can be cross linked gel, entangled polymer or a buffer, for example. The buffers for the second separation can be, for example, contained in buffer reservoirs (125). These buffers can be liquid, or can be embedded in a gel. Two different buffers can be utilized if desired to create discontinuous separation for higher resolution.

Two dimensional separation can also be accomplished by utilizing the capture and release method and a single second dimension channel. In this case the first captured fraction will be separated in the second dimension, and then the subsequent released fractions will be separated. The separation can be used for analytical purposes or harvesting ports can be incorporated in the channels to allow the harvesting of the separated analytes if needed.

### Methods and devices for purifying a target molecule using pI focusing and subsequent crystallization

Crystallography is used to analyze the structure of proteins. This is a very valuable technique, however also very challenging due to the high requirements for protein purity. Typically the protein is purified to more than 90% pure and is concentrated to about 10 mg/ml. The crystallization process is performed at the pH = pI of the protein. The typical purification process is challenging and frequently 2 to 5 different separation steps are used in order to achieve high purity. After that the protein is usually concentrated using a molecular weight cutoff membrane. An example of protein crystallization and x-ray defraction can be found in, Yamano A, et al., J Biol Chem. 272 (15): 9597-600 (1997).

Disclosed herein are proton injector and/or hydroxide injector-based methods for purifying proteins for crystallization. Proton/hydroxide injector technology may be used to focus the target protein at its pI. This can be done as part of, or as the last or penultimate step in the purification workflow, e.g., prior to crystallization. The proton/hydroxide injector step can be combined as the last purification and concentration step. The proton/hydroxide injector step may provide an additional purification step orthogonal to the chromatography steps typically used and in the same time can concentrate the protein to very high degree essentially eliminating the need for separate concentration step.

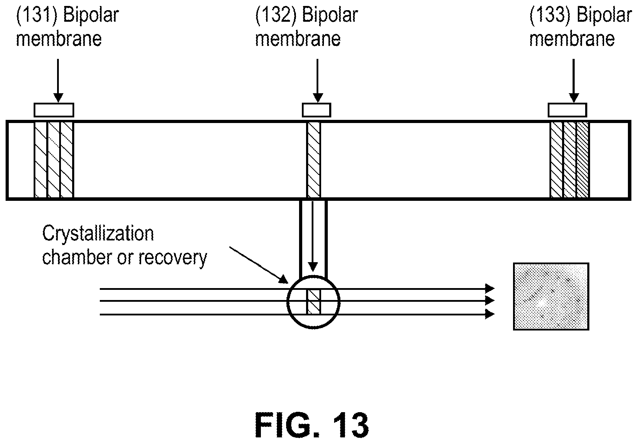

Figure 13 illustrates the bipolar membrane (131) creating a pH step above the pH of the target protein, therefore trapping all proteins with pI higher than the pI of the target protein. A second proton injector or hydroxide injector separated from the channel by a second bipolar membrane (132) creates a very narrow pH step at the pI of the target protein therefore capturing and concentrating it at this position. A third proton injector or hydroxide injector separated from the channel by a third bipolar membrane (133) creates a pH step below the pI of the target protein therefore capturing all proteins with pI below the pI of the target protein. Once captured and focused in very sharp boundary and therefore highly concentrated, the target protein can either be moved electrophoretically, or by using liquid flow, to a place where the protein can be recovered or stored for crystallization and imaged with X-ray directly in the microfluidic cartridge. As shown in Figure 6 it may be possible to capture and work with multiple proteins at the same time. An array can be used to crystallize multiple proteins at once or to test multiple conditions for the same protein.

The term sample relates to any type of sample, including a biological sample. "Biological sample" encompasses a variety of sample types obtained from an organism. The term encompasses bodily fluids such as blood, saliva, serum, plasma, urine and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, sedimentation, or enrichment for certain components. The term encompasses a clinical sample, and also includes cells in cell culture, cell supernatants, cell lysates, serum, plasma, other biological fluids, and tissue samples.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer. Peptides can be of any length of two or more amino acids, e.g., 6-100, 80-50 and 10-40 amino acids.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Examples not covered by the scope of the claims are for illustrative purposes.

### EXAMPLE

### Example 1: Separation of Target(s) Based on pI

Two fluorescently-labeled peptides, one with a pI of 5.0, one with a pI of 6.8, were placed into a chamber comprising a pH 8.5 phosphate buffer. The chamber comprises two proton injectors, with the first proton injector having a current applied of 150 µA and the second proton injector having a current applied of 65 µA, thereby generating separate localized areas within the solution having different pH. In view of the higher current, the first injector generated a more acidic pH in the area of the chamber near the first injector compared to the pH near the second injector. An electric field was generated across the chamber, thereby moving charged molecules according to their charge. The pI 6.8 peptides focused on the area near the first proton injector and the pI 5.0 peptides focused on the area of the chamber near the second proton injector. This shows that molecules having different pI can be moved and isolated in different areas of a solution in a chamber using electronic control of their movement in combination with localization based on control of local pH in the solution using ion injectors.

### Example 2: Precipitation/Trapping of Target(s) Based on pI

This experiment shows that some target molecules precipitate or adhere to channel surface when positioned at their pI under prolonged H⁺ injection, and that the resulting targets can subsequently be immuno-detected. Green Fluorescent Protein (GFP, 1 µg) and human saliva (1.5 µg) were combined with STB 8.5 (4 mM each Sodium Citrate, Sodium Phosphate, Sodium Pyrophosphate, and 13 mM Sodium Sulfate, pH 8.5) and the resulting mixture was introduced into a chamber comprising a proton injector. The injector was set to generate a pH step encompassing the pI of GFP (∼5.4) and voltage was run through the first and second electrodes across the chamber, thereby electrophoresing GFP through the chamber and up to the pH gradient, where GFP stopped due to lack of charge. GFP was 'trapped' following prolonged H⁺ injection (>15 min) after isolectric focusing over a bipolar membrane (BPM).

While not necessarily true for all targets, GFP precipitated/adhered to channel surface following prolonged H⁺ injection. The H⁺ injection current was subsequently turned off. As shown in Figure 19A and 20A, which detects GFP fluorescence, GFP localized at the pH step. Subsequently, an anti-GFP antibody labeled with Dyelight649/DL649 was introduced to the chamber and electrophoresed for 60 min across channel and over the GFP precipitate. Signal under a Cy5 filter, which also detects DL649 fluorescence, shows that the anti-GFP antibody localized with the GFP (Figure 19B), demonstrating that this system detects target molecules that are localized in a pH step gradient. In contrast, Figure 20B displays results from a parallel experiment using a non-specific anti-rabbit antibody. Only background signal was observed from the non-specific antibody.

## Claims

1. A device for separating a plurality of molecular analytes according to both isoelectric points and electrophoretic mobility within one dimension, the device comprising
a chamber for containing a solution having a plurality of molecular analytes, wherein the chamber contains a sieving medium suitable for electrophoresis;
an electrical source for applying an electric field along an axis in the chamber;
a one or more ion sources for establishing a pH gradient along said axis in said chamber by injecting ion flows, capable of forming one or more pH steps in a pH gradient, wherein each of said ion sources is a proton injector or a hydroxide injector separated from said chamber by a bipolar membrane; and
a controller which operates said one or more ion sources to adjust the pH gradient so as to induce migration and capturing of the molecular analytes separately along the axis.

2. The device of claim 1, wherein the chamber contains one or more ports in fluid communication with the chamber and movably positioned in the chamber to capture a desired analyte based on the analyte's position.

3. A method of separating one or more target protein from a sample, the method comprising,
providing into the chamber of the device of claim 1 or 2 a sample comprising a mixture of proteins including one or more target protein;
submitting the proteins in the chamber to electrophoresis; and
subsequently generating a pH gradient in the chamber with a proton and/or hydroxide injector, thereby positioning proteins in the chamber within one dimension based on both the isoelectric point (pI) and the electrophoretic mobility of the proteins.

4. The method of claim 3, further comprising collecting the one or more target protein.

## Patentansprüche

1. Vorrichtung zum Trennen einer Vielzahl von molekularen Analyten sowohl nach ihren isoelektrischen Punkten als auch ihrer elektrophoretischen Mobilität innerhalb einer einzigen Dimension, wobei die Vorrichtung umfasst:
eine Kammer zum Aufnehmen einer Lösung, die eine Vielzahl von molekularen Analyten aufweist, wobei die Kammer ein für die Elektrophorese geeignetes Siebmedium enthält;
eine Stromquelle zum Anlegen eines elektrischen Felds entlang einer Achse in der Kammer;
eine oder mehrere Ionenquellen zum Aufbauen eines pH-Gradienten entlang der Achse in der Kammer durch Injizieren von Ionenströmen, die in einem pH-Gradienten eine oder mehrere pH-Stufen bilden können, wobei jede der Ionenquellen ein Protoneninjektor oder ein Hydroxidinjektor ist, die durch eine bipolare Membran von der Kammer getrennt sind; und
eine Steuervorrichtung, die die eine oder die mehreren Ionenquellen so betreibt, dass der pH-Gradient eingestellt wird, um eine Migration und ein getrenntes Auffangen der molekularen Analyten entlang der Achse zu induzieren.

2. Vorrichtung gemäß Anspruch 1, wobei die Kammer einen oder mehrere Anschlüsse in Fluidkommunikation mit der Kammer enthält, die in der Kammer beweglich positioniert sind, um einen gewünschten Analyten auf der Basis der Position des Analyten abzufangen.

3. Verfahren zum Abtrennen von einem oder mehreren Zielproteinen aus einer Probe, wobei das Verfahren umfasst:
Bereitstellen einer Probe, die ein Gemisch von Proteinen umfasst, das ein oder mehrere Zielproteine umfasst, in der Kammer der Vorrichtung gemäß Anspruch 1 oder 2;
Durchführen einer Elektrophorese mit den Proteinen in der Kammer; und
anschließend Erzeugen eines pH-Gradienten in der Kammer mit einem Protonen- und/oder Hydroxidinjektor, wodurch Proteine in der Kammer innerhalb einer Dimension sowohl anhand ihres isoelektrischen Punkts (pI) als auch anhand der elektrophoretischen Mobilität der Proteine positioniert werden.

4. Verfahren gemäß Anspruch 3, weiterhin umfassend das Auffangen des einen oder der mehreren Zielproteine.

## Revendications

1. Dispositif de séparation d'une pluralité d'analytes moléculaires selon à la fois les points isoélectriques et la mobilité électrophorétique dans une dimension, ledit dispositif comprenant :
une chambre pouvant contenir une solution renfermant une pluralité d'analytes moléculaires, dans lequel la chambre contient un milieu de tamisage approprié pour l'électrophorèse ;
une source électrique pour appliquer un champ électrique le long d'un axe dans ladite chambre ;
une ou plusieurs sources d'ions pour établir un gradient de pH le long dudit axe dans ladite chambre par injection de flux ioniques, susceptibles de former une ou plusieurs étapes de pH dans un gradient de pH, dans lequel chacune desdites sources d'ions est un injecteur de protons ou un injecteur d'hydroxyde séparé de ladite chambre par une membrane bipolaire ; et
un dispositif de commande qui contrôle ladite ou lesdites sources d'ions pour ajuster le gradient de pH de manière à induire la migration et la capture des analytes moléculaires séparément le long de l'axe.

2. Dispositif selon la revendication 1, dans lequel la chambre contient un ou plusieurs orifices en communication fluidique avec la chambre et positionnés de manière mobile dans la chambre pour capturer un analyte souhaité en fonction de la position de ce dernier.

3. Procédé de séparation d'une ou de plusieurs protéines cibles à partir d'un échantillon, ledit procédé comprenant les étapes consistant à :
introduire dans la chambre du dispositif selon la revendication 1 ou 2 un échantillon comprenant un mélange de protéines renfermant une ou plusieurs protéines cibles ;
soumettre les protéines dans la chambre à une électrophorèse ; et
ensuite générer un gradient de pH dans la chambre à l'aide d'un injecteur de protons et/ou d'hydroxyde, positionnant ainsi les protéines dans la chambre dans une dimension en fonction à la fois de leur point isoélectrique (pI) et de leur mobilité électrophorétique.

4. Procédé selon la revendication 3, comprenant en outre la collecte de la ou des protéines cibles.
